# EUROPÄISCHE PATENTSCHRIFT

(11) **EP 2 461 889 B1**
(45) Veröffentlichungstag und Bekanntmachung des Hinweises auf die Patenterteilung: **04.09.2013**
(21) Anmeldenummer: 10742125.7
(22) Anmeldetag: 03.08.2010
(51) Int. Cl.: B01D 53/14

(54) **ABSORPTIONSMITTEL ZUM ENTFERNEN SAURER GASE AUS EINEM FLUIDSTROM**
ABSORPTION AGENT FOR REMOVING ACIDIC GASES FROM A FLUID FLOW
AGENT D'ABSORPTION POUR L'ÉLIMINATION DE GAZ ACIDES À PARTIR D'UN COURANT FLUIDE

(30) Priorität: 04.08.2009 EP 09167181
(43) Veröffentlichungstag der Anmeldung: 13.06.2012
(73) Patentinhaber: BASF SE, 67056 Ludwigshafen (DE)
(72) Erfinder: RIEMANN, Christian, 67122 Altrip (DE); KATZ, Torsten, 67434 Neustadt (DE); SIEDER, Georg, 67098 Bad Dürkheim (DE); VORBERG, Gerald, 67346 Speyer (DE); DENGLER, Erika, 67459 Böhl-Iggelheim (DE)
(86) Internationale Anmeldenummer: PCT/EP2010/061243
(87) Internationale Veröffentlichungsnummer: WO 2011/015565

(56) Entgegenhaltungen:
- EP-A2- 0 671 200
- WO-A1-2008/072979

## Beschreibung

Die vorliegende Erfindung betrifft ein Absorptionsmittel und ein Verfahren zum Entfernen saurer Gase aus einem Fluidstrom, insbesondere einem sauerstoffhaltigen Fluidstrom.

Die Entfernung saurer Gase, wie z.B. CO₂, H₂S, SO₂, CS₂, HCN, COS oder Merkaptanen, aus Fluidströmen ist aus unterschiedlichen Gründen von Bedeutung.

Die Entfernung von Kohlendioxid aus Verbrennungsabgasen bzw. Rauchgasen ist aus verschiedenen Gründen wünschenswert, insbesondere aber zur Verminderung der Emission von Kohlendioxid, die als Hauptursache für den so genannten Treibhauseffekt angesehen wird.

Die Entfernung von Kohlendioxid und Schwefelwasserstoff aus Biogas dient der Methananreicherung, um das Biogas auf Erdgasqualität aufzubereiten.

Der Gehalt an Schwefelverbindungen von Erdgas muss durch geeignete Aufbereitungsmaßnahmen unmittelbar an der Erdgasquelle reduziert werden, denn die Schwefelverbindungen bilden in dem vom Erdgas häufig mitgeführten Wasser Säuren, die korrosiv wirken. Für den Transport des Erdgases in einer Pipeline müssen daher vorgegebene Grenzwerte der schwefelhaltigen Verunreinigungen eingehalten werden. Die Verringerung des Gehalts an Kohlendioxid ist vielfach zur Einstellung eines vorgegebenen Brennwerts erforderlich.

Zur Entfernung von sauren Gasen werden häufig Wäschen mit Absorptionsmitteln in Form wässriger Lösungen organischer Amine eingesetzt. Beim Lösen saurer Gase in dem Absorptionsmittel bilden sich mit den Aminen Ionen. Das Absorptionsmittel kann durch Entspannen auf einen niedrigeren Druck und/oder Strippen regeneriert werden, wobei die ionischen Spezies zu sauren Gasen zurück reagieren und/oder mittels Dampf abgestrippt werden. Nach dem Regenerationsprozess kann das Absorptionsmittel wiederverwendet werden.

Insbesondere bei Wäschen, die bei niedrigem Druck nahe dem Normaldruck durchgeführt werden, können Amine, die einen vergleichsweise hohen Dampfdruck aufweisen, in das Reingas übertreten. Zur Vermeidung unerwünschter Emissionen muss das nach der Amin-Wäsche erhaltene Reingas einer weiteren Wäsche mit Wasser unterzogen werden.

Bestimmte Probleme treten bei der Behandlung sauerstoffhaltiger Fluide, z. B. von Rauchgasen, auf. Hierbei verschlechtert sich die Absorptionsfähigkeit des Absorptionsmittels langfristig und wird bei der Regeneration nicht vollständig wiedererlangt. Vermutlich ist die Gegenwart von molekularem Sauerstoff für eine oxidative Zersetzung der im Absorptionsmittel enthaltenen Amine verantwortlich. Zur Vermeidung dieses Problems ist bereits vorgeschlagen worden, dem Absorptionsmittel Stabilisatoren gegen die Sauerstoff-induzierte Zersetzung zuzusetzen. Obgleich die Verwendung von Stabilisatoren die Zersetzung der Amine wirksam unterdrückt, ist ihr Einsatz mit erheblichen Kosten verbunden, da die Stabilisatormenge laufend ergänzt werden muss.

Die EP-A 671 200 beschreibt ein Verfahren zur Entfernung von Kohlendioxid aus Verbrennungsabgasen, bei dem das Verbrennungsabgas mit einer wässrigen Lösung eines Aminosäuremetallsalzes und Piperazin in Kontakt bringt. Veranschaulichte Aminosäuremetallsalze sind Kalium-dimethylaminoacetat und Kalium-α-methylaminopropionat.

Die EP-A 1 806 171 offenbart ein Verfahren zur Gewinnung von SO₂ und CO₂ aus einem Gasstrom. Es kommt ein Absorptionsmittel zum Einsatz, das wenigstens ein tertiäres Amin und wenigstens ein sekundäres Amin als Aktivator enthält. Beispielhafte Aktivatoren sind N-Hydroxyethylpiperazin, Piperazin und N-Hydroxypropypiperazin.

Die US 2004/0253159 offenbart ein Verfahren zur Gewinnung von CO₂ aus einem Gasstrom. Es kommt ein Absorptionsmittel zum Einsatz, das wenigstens ein tertiäres Amin mit einem pKa von 6,5 bis 9 aufweist. Das Absorptionsmittel enthält fakultativ ein sekundäres Amin. Beispielhafte sekundäre Amine sind N-Hydroxyethylpiperazin, Piperazin und N-Hydroxypropypiperazin.

Die DE 103 06 254 beschreibt ein Absorptionsmittel zur Entfernung von sauren Gasen aus Fluiden, das wenigstens ein tertiäres Alkanolamin und ein Amin umfasst, das ausgewählt ist unter Hydroxyethylpiperazin, Bis(hydroxyethyl)piperazin oder einem Gemisch davon.

Die WO 2007/135028 beschreibt ein Verfahren zum Entfernen saurer Gase aus einem kohlenwasserstoffhaltigen Fluidstrom oder einem sauerstoffhaltigen Fluidstrom, bei dem man den Fluidstrom mit einer wässrigen Lösung in Kontakt bringt, die wenigstens ein Amin und wenigstens ein Metallsalz einer Aminocarbonsäure und/oder Aminosulfonsäure umfasst.

Die WO 2008/072979 A1 offenbart ein Verfahren zum Abfangen von CO₂ aus Verbrennungsabgas, wobei das CO₂-haltige Gas durch eine wässrige Aufschlämmung eines Absorptionsmittels geführt wird. Die wässrige Aufschlämmung umfasst ein anorganisches Alkalicarbonat, -bicarbonat und zumindest einen Absorptionspromotor und/oder Katalysator. Als Absorptionspromotor und/oder Katalysator sind unter anderem N-2-Hydroxyethylpiperazin, Glycin und Sarkosin beschrieben.

Der Erfindung liegt die Aufgabe zugrunde, ein Absorptionsmittel zum Entfernen saurer Gase aus einem Fluidstrom anzugeben, dessen von Wasser verschiedene Komponenten einen niedrigen Dampfdruck aufweisen und das eine erhöhte Beständigkeit gegenüber Sauerstoff aufweist.

Die Aufgabe wird gelöst durch ein Absorptionsmittel zum Entfernen saurer Gase aus einem Fluidstrom, das eine wässrige Lösung
(A) eines Alkalimetallsalzes einer N,N-Di-C₁-C₄-alkyl-aminocarbonsäure und
(B) N-Hydroxyethylpiperazin
umfasst.

Die Erfindung betrifft außerdem ein Verfahren zum Entfernen saurer Gase aus einem Fluidstrom, bei dem man den Fluidstrom mit dem vorstehend definierten Absorptionsmittel in Kontakt bringt.

Das Alkalimetallsalz der N,N-Di-C₁-C₄-alkyl-aminocarbonsäure verfügt über eine tertiäre Aminogruppe. Während primäre und sekundäre Amine mit Kohlendioxid lösliche Carbamate bilden, reagieren tertiäre Aminogruppen nicht direkt mit Kohlendioxid, da das Stickstoffatom vollständig substituiert ist. Vielmehr wird Kohlendioxid mit dem tertiären Amin und mit Wasser zu Bicarbonat in einer Reaktion mit geringer Reaktionsrate umgesetzt. Da keine direkte Bindung zwischen tertiären Aminen und Kohlendioxid entsteht, kann die Aminlösung sehr wirtschaftlich regeneriert werden. Nachteilig an der Verwendung von tertiären Aminlösungen ist allerdings, dass wegen der geringen Reaktionsrate des Kohlendioxids der Waschprozess mit einer sehr hohen Verweilzeit durchgeführt werden muss. Die Absorptionsrate von Kohlendioxid in wässrigen Lösungen von tertiären Aminen kann durch Zugabe weiterer Verbindungen, die als Aktivatoren oder Promotoren bezeichnet werden, erhöht werden. Primäre oder sekundäre Amine sind geeignete Aktivatoren. Im erfindungsgemäßen Absorptionsmittel kommt dem N-Hydroxyethylpiperazin die Funktion eines Aktivators zu.

Das erfindungsgemäße Absorptionsmittel mit N-Hydroxyethylpiperazin als Aktivator erzielt vergleichbare Absorptionsleistungen wie Vergleichsabsorptionsmittel mit gleichen molaren Konzentrationen an Piperazin. Es wurde aber gefunden, dass N-Hydroxyethylpiperazin in Gegenwart von Sauerstoff unerwartet stabil ist, während sich Piperazin, das wie N-Hydroxyethylpiperazin über sekundäre Aminogruppe(n) verfügt, in Gegenwart von Sauerstoff rasch zersetzt.

Das Alkalimetallsalz der N,N-Di-C₁-C₄-alkyl-aminocarbonsäure weist als salzartige Verbindung praktisch keinen Dampfdruck auf. Es weist eine ausgezeichnete Stabilität gegenüber Sauerstoff auch bei erhöhten Temperaturen auf.

Die wässrige Lösung umfasst im Allgemeinen 2 bis 5 kmol / m³, vorzugsweise 2,3 bis 3,3 kmol / m³, Alkalimetallsalz der N,N-Di-C₁-C₄-alkyl-aminocarbonsäure; sie umfasst im Allgemeinen 0,1 bis 1,5 kmol / m³, vorzugsweise 0,5 bis 1,2 kmol / m³, N-Hydroxyethylpiperazin.

Aminocarbonsäuren enthalten wenigstens eine Aminogruppe und wenigstens eine Carboxylgruppe in ihrer Molekülstruktur. Wenn die Aminocarbonsäure ein oder mehrere chirale Kohlenstoffatome aufweist, ist die Konfiguration unbeachtlich; es können sowohl die reinen Enantiomere/Diastereomere als auch beliebige Gemische oder Racemate verwendet werden.

Die Aminocarbonsäure ist vorzugsweise eine α-Aminosäure oder eine β-Aminosäure. Davon sind α-Aminosäuren besonders bevorzugt. Die Bezeichnung "α" bzw. "β" bedeutet in Übereinstimmung mit der üblichen Nomenklatur, dass die Aminogruppe durch ein bzw. zwei Kohlenstoffatome von der Carboxylgruppe getrennt ist.

Geeignete Alkalimetallsalze einer N,N-Di-C₁-C₄₋alkyl-aminocarbonsäure sind beispielsweise Alkalimetallsalze von N,N-Dimethylglycin (Dimethylaminoessigsäure), N,N-Diethylclycin, N,N-Dimethylalanin, N,N-Dimethylleucin, N,N-Dimethylisoleucin, N,N-Dimethylvalin und N,N-Dimethylserin.

Das Alkalimetallsalz ist im Allgemeinen ein Natrium- und/oder Kaliumsalz, vorzugsweise ein Kaliumsalz.

Ein bevorzugtes Alkalimetallsalz einer N,N-Di-C₁-C₄₋alkyl-aminocarbonsäure ist Kalium-N,N-dimethylglycinat.

Das Absorptionsmittel kann auch Additive, wie Korrosionsinhibitoren, Enzyme etc. enthalten. Im allgemeinen liegt die Menge an derartigen Additiven im Bereich von etwa 0,01-3 Gew.-% des Absorptionsmittels.

Zu den sauren Gasen, die mit dem erfindungsgemäßen Absorptionsmittel entfernt werden können zählen Kohlendioxid, H₂S, SO₃, SO₂, CS₂, HCN, COS, Disulfide und Mercaptane. In der Regel umfassen die sauren Gase zumindest Kohlendioxid und gegebenenfalls weitere saure Gase. So können die zu entfernenden sauren Gase z. B. Kohlendioxid und H₂S oder Kohlendioxid und SO₂ umfassen.

Das erfindungsgemäße Verfahren bzw. Absorptionsmittel ist geeignet zur Behandlung von Fluiden aller Art. Fluide, welche die sauren Gase enthalten, sind einerseits Gase, wie Erdgas, Synthesegas, Koksofengas, Spaltgas, Kohlevergasungsgas, Kreisgas, Deponiegase und Verbrennungsabgase, und andererseits mit dem Absorptionsmittel im Wesentlichen nicht mischbare Flüssigkeiten, wie LPG (Liquefied Petroleum Gas) oder NGL (Natural Gas Liquids).

Aufgrund der hervorragenden Beständigkeit des erfindungsgemäßen Absorptionsmittels eignet es sich besonders zur Entfernung saurer Gase aus sauerstoffhaltigen Fluidströmen. Der Sauerstoffgehalt derartiger Fluidströme beträgt üblicherweise 0,01 bis 15 Vol.-%, vorzugsweise 0,1 bis 10 Vol.-%.

Bei dem sauerstoffhaltigen Fluidstrom handelt es sich z. B. um einen Gasstrom, der durch Oxidation organischer Substanzen gebildet ist. Die Oxidation kann unter Flammenerscheinung, d. h. als herkömmliche Verbrennung, oder als Oxidation ohne Flammenerscheinung, z. B. in Form einer katalytischen Oxidation oder Partialoxidation, durchgeführt werden. Organische Substanzen, die der Verbrennung unterworfen werden, sind üblicherweise fossile Brennstoffe wie Kohle, Erdgas, Erdöl, Benzin, Diesel, Raffinate oder Kerosin, Biodiesel oder Abfallstoffe mit einem Gehalt an organischen Substanzen. Ausgangsstoffe der katalytischen (Partial-) Oxidation sind z. B. Methanol oder Methan, das zu Ameisensäure oder Formaldehyd umgesetzt werden kann. Die Verbrennung der organischen Substanzen erfolgt meistens in üblichen Verbrennungsanlagen mit Luft.

Bevorzugte sauerstoffhaltige Fluidströme sind Verbrennungsabgase.

Das Verfahren eignet sich auch zur Behandlung der Abgase von Brennstoffzellen oder chemischer Syntheseanlagen, die sich einer (Partial-) Oxidation organischer Substanzen bedienen.

Das erfindungsgemäße Verfahren bzw. Absorptionsmittel ist ferner zur Behandlung von kohlenwasserstoffhaltigen Fluidströmen geeignet. Die enthaltenen Kohlenwasserstoffe sind z. B. aliphatische Kohlenwasserstoffe, wie C₁-C₄-Kohlenwasserstoffe, wie Methan, ungesättigte Kohlenwasserstoffe, wie Ethylen oder Propylen, oder aromatische Kohlenwasserstoffe wie Benzol, Toluol oder Xylol.

Ein kohlenwasserstoffhaltiger Fluidstrom, der mit dem erfindungsgemäßen Absorptionsmittel behandelt werden kann, ist Biogas. Biogas ist ein brennbares Gas, das durch Vergärung, d. h. im Wesentlichen anaerobe Zersetzung, von Biomasse hergestellt wird.

Der typische Abbauprozess von organischem Material zu Biogas besteht im Wesentlichen aus vier Stufen. In der ersten Stufe (Hydrolyse) bauen aerobe Bakterien die hochmolekularen organischen Substanzen (Eiweiß, Kohlenhydrate, Fett, Zellulose) mit Hilfe von Enzymen in niedermolekulare Verbindungen wie Einfachzucker, Aminosäuren, Fettsäuren und Wasser um. Die von den hydrolytischen Bakterien ausgeschiedenen Enzyme haften an der Außenseite der Bakterien (so genannte Exoenzyme) und spalten dabei die organischen Bestandteile des Substrats hydrolytisch in kleine wasserlösliche Moleküle auf. In der zweiten Stufe (Versäuerung) werden die einzelnen Moleküle intrazellulär von säurebildenden Bakterien abgebaut und umgewandelt. Es handelt sich dabei um fakultativ aerobe Spezies, die den noch verbliebenen Sauerstoff weitgehend verbrauchen und so die für die Methanbakterien nötigen anaeroben Bedingungen schaffen. Hier werden hauptsächlich kurzkettige Fettsäuren, niedermolekulare Alkohole und Gase erzeugt. In der dritten Stufe (Essigsäurebildung) produzieren Essigsäurebakterien aus den organischen Säuren die Ausgangsprodukte für die Methanbildung (Essigsäure, Kohlendioxid und Wasserstoff). In der vierten Stufe (Methanbildung) bilden Methanbakterien das Methan.

Vor der Aufbereitung ist Biogas eine Gasmischung mit den Hauptkomponenten Methan und Kohlendioxid. In geringen Mengen sind meist auch Stickstoff, Sauerstoff, Schwefelwasserstoff, Wasserstoff und Ammoniak enthalten. Eine typische Zusammensetzung von Biogas ist: Methan 45 - 70 Vol.-%, Kohlendioxid 25 - 55 Vol.-%, Wasserdampf 0 - 10 Vol.-%, Stickstoff 0,01 - 5 Vol.-%, Sauerstoff 0,01 - 2 Vol.-%, Wasserstoff 0 - 1 Vol.-%, Ammoniak 0,01 - 2,5 mg/m³, Schwefelwasserstoff 10 - 10.000 mg/m³. Aufgrund der hervorragenden Beständigkeit des erfindungsgemäßen Absorptionsmittels gegenüber Sauerstoff eignet es sich besonders zur Entfernung saurer Gase aus Biogas mit einem Gehalt an Sauerstoff, z. B. einem Sauerstoffgehalt von 0,01 bis 2 Vol.-%.

Als Biomasse werden üblicherweise Stalldung, Stroh, Jauche, Klärschlamm, Fermentationsrückstände und dergleichen verwendet. In Betracht kommen auch stärkehaltige Kornfrüchte oder Samen. Die bakterielle Zersetzung erfolgt z. B. in üblichen Biogasanlagen. Die Beschickung eines Biogasreaktors kann kontinuierlich oder diskontinuierlich erfolgen. Bei der diskontinuierlichen Beschickung, dem so genannten Batch-Prinzip, wird der ganze Faulbehälter auf einmal befüllt. Die Charge fault ohne Substratwechsel bis zum Ende der gewählten Verweilzeit. Dabei setzt die Gasproduktion nach der Füllung ein, erreicht ein Maximum und flacht dann ab. Nach Ablauf der Verweilzeit wird der Behälter bis auf einen als Impfmaterial wirkenden Rest für die nächste Charge vollständig entleert. Die ungleichmäßige Gasproduktion kann durch mehrere kleinere Fermenter, die phasenversetzt befüllt werden, ausgeglichen werden. Mehrere kleinere Behälter verursachen jedoch höhere spezifische Kosten. Vorteilhaft sind Fermenter, die kontinuierlich mit den zu vergärenden Stoffen beschickt werden, wobei gleichzeitg eine entsprechende Menge von ausgefaultem Substrat abgepumpt wird. Dadurch wird eine permanent stattfindende Faulung mit einer konstanten Gasproduktion erreicht und außerdem einer Versäuerung durch die häufige Zugabe kleiner Substratmengen vorgebeugt.

Daneben kann das erfindungsgemäße Verfahren natürlich auch angewendet werden, um unverbrannte fossile Gase, wie Erdgas, z. B. so genannte Coal-seam-Gase, d. h. bei der Förderung von Kohle anfallende Gase; die gesammelt und komprimiert werden, zu behandeln.

Zur Durchführung des erfindungsgemäßen Verfahrens geeignete Vorrichtungen umfassen wenigstens eine Waschkolonne, z. B. Füllkörper, Packungs- und Bodenkolonnen, und/oder andere Absorber wie Membrankontaktoren, Radialstromwäscher, Strahlwäscher, Venturi-Wäscher und Rotations-Sprühwäscher. Die Behandlung des Gasstroms mit dem Absorptionsmittel erfolgt dabei bevorzugt in einer Waschkolonne im Gegenstrom. Der Gasstrom wird dabei im Allgemeinen in den unteren Bereich und das Absorptionsmittel in den oberen Bereich der Kolonne eingespeist.

Die Temperatur des Absorptionsmittels beträgt im Absorptionsschritt im Allgemeinen etwa 30 bis 70°C, bei Verwendung einer Kolonne beispielsweise 30 bis 60°C am Kopf der Kolonne und 40 bis 70°C am Boden der Kolonne. Es wird ein an sauren Gasbestanteilen armes, d. h. ein an diesen Bestandteilen abgereichertes Produktgas (Beigas) und ein mit sauren Gasbestandteilen beladenes Absorptionsmittel erhalten.

Das erfindungsgemäße Verfahren eignet sich besonders zur Behandlung von Fluidströmen, die bei einem Druck nahe dem Normaldruck anfallen, wie z. B. Verbrennungsabgase oder Biogas, und zur Behandlung nicht wesentlich verdichtet werden. Aufgrund des niedrigen Dampfdrucks des Absorptionsmittels treten keine nennenswerten Mengen an Absorptionsmittelkomponenten in den behandelten Fluidstrom über und eine Wäsche des behandelten Fluidstroms ist entbehrlich. In bevorzugten Ausführungsformen erfolgt das Inkontaktbringen des Fluidstroms mit dem Absorptionsmittel bei einem Druck von 1,0 bis 3,0 bar (absoluter Druck).

Aus dem mit den sauren Gasbestandteilen beladenen Absorptionsmittel kann das Kohlendioxid in einem Regenerationsschritt freigesetzt werden, wobei ein regeneriertes Absorptionsmittel erhalten wird. Im Regenerationsschritt wird die Beladung des Absorptionsmittels verringert und das erhaltene regenerierte Absorptionsmittel wird vorzugsweise anschließend in den Absorptionsschritt zurückgeführt.

Im Allgemeinen regeneriert man das beladene Absorptionsmittel durch
a) Erwärmung, z. B. auf 70 bis 130°C,
b) Entspannung,
c) Strippen mit einem inerten Fluid
oder eine Kombination zweier oder aller dieser Maßnahmen.

In der Regel wird das beladene Absorptionsmittel zur Regeneration erwärmt und das freigesetzte Kohlendioxid wird z. B. in einer Desorptionskolonne abgetrennt. Bevor das regenerierte Absorptionsmittel wieder in den Absorber eingeführt wird, wird es auf eine geeignete Absorptionstemperatur abgekühlt. Um die im heißen regenerierten Absorptionsmittel enthaltene Energie auszunutzen, ist es bevorzugt, das beladene Absorptionsmittel aus dem Absorber durch Wärmetausch mit dem heißen regenerierten Absorptionsmittel vorzuerwärmen. Durch den Wärmetausch wird das beladene Absorptionsmittel auf eine höhere Temperatur gebracht, so dass im Regenerationsschritt ein geringerer Energieeinsatz erforderlich ist. Durch den Wärmetausch kann auch bereits eine teilweise Regenerierung des beladenen Absorptionsmittels unter Freisetzung von Kohlendioxid erfolgen. Der erhaltene gas-flüssig-gemischtphasige Strom wird in ein Phasentrenngefäß geleitet, aus dem das Kohlendioxid abgezogen wird; die Flüssigphase wird zur vollständigen Regeneration des Absorptionsmittels in die Desorptionskolonne geleitet.

Die Erfindung wird anhand der beigefügten Figur und des nachfolgenden Beispiels näher erläutert.

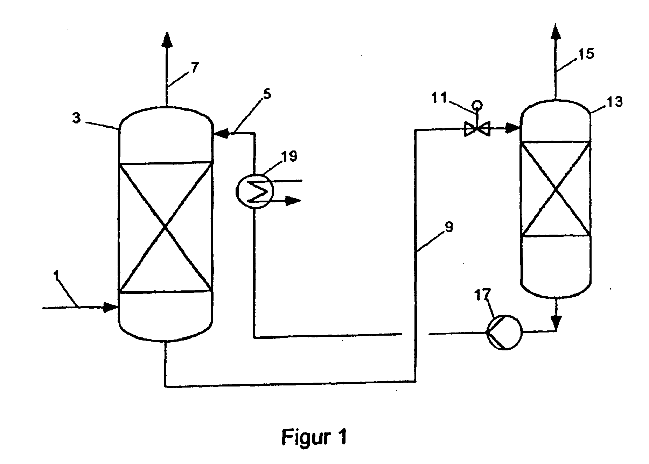

Fig. 1 ist eine schematische Darstellung einer zur Durchführung des erfindungsgemäßen Verfahrens geeigneten Anlage.

Gemäß Fig. 1 wird über eine Zuleitung 1 ein geeignet vorbehandeltes, Kohlendioxid enthaltendes Verbrennungsgas in einem Absorber 3 mit dem regenerierten Absorptionsmittel, das über die Absorptionsmittelleitung 5 zugeführt wird, im Gegenstrom in Kontakt gebracht. Das Absorptionsmittel entfernt Kohlendioxid durch Absorption aus dem Verbrennungsgas; dabei wird über eine Abgasleitung 7 ein an Kohlendioxid armes Reingas gewonnen. Über eine Absorptionsmittelleitung 9 und ein Drosselventil 11 wird das mit Kohlendioxid beladene Absorptionsmittel einer Desorptionskolonne 13 zugeleitet. Im unteren Teil der Desorptionskolonne 13 wird das beladene Absorptionsmittel mittels eines (nicht dargestellten) Aufheizers erwärmt und regeneriert. Das dabei freigesetzte Kohlendioxid verlässt die Desorptionskolonne 13 über die Abgasleitung 15. Das regenerierte Absorptionsmittel wird anschließend mittels einer Pumpe 17 über einen Wärmetauscher 19 der Absorptionskolonne 3 wieder zugeführt.

### Beispiel

Ein Vergleichs-Absorptionsmittel bestand aus einer wässrigen Lösung von Kalium-N,N-dimethylglycinat (7 mol-%) und Piperazin (2 mol-%). Ein erfindungsgemäßes Lösungsmittel bestand aus einer wässrige Lösung von Kalium-N,N-dimethylglycinat (7 mol-%) und N-Hydroxyethyl-piperazin (2 mol-%). Beide Absorptionsmittel wurden folgendem Stresstest unterzogen: Das wässrige Absorptionsmittel wurde in einen Glaskolben gefüllt. Der Glaskolben wurde von unten beheizt und auf 100°C Siedetemperatur gehalten. Auf dem Glaskolben ist ein Gaskühler aufgesetzt, der die Gasphase auf etwa 4°C kühlt. Dabei schlägt sich die im Gasstrom befindliche Aminphase flüssig nieder und fließt zurück in den Glaskolben. Über einen weiteren Zugang am Glaskolben wird ein Gasgemischstrom von 10 Litem/h, bestehend aus 9 Anteilen Luft und einem Anteil CO₂, in das Lösungsmittel eingeleitet.

Nach dem 28. Tag wurden Aliquote der Absorptionsmittel entnommen und der Gehalt an Piperazin bzw. N-Hydroxyethyl-piperazin mittels gaschromatographischer Analyse bestimmt. Die Wiederfindungsmengen an Piperazin bzw. N-Hydroxyethyl-piperazin sind in der nachstehenden Tabelle dargestellt. Man sieht, dass Piperazin im Verlauf des Tests deutlich stärker abgebaut wird als N-Hydroxyethyl-piperazin, das selbst nach 4 Wochen nahezu vollständig wiedergefunden wird. N-Hydroxyethyl-piperazin zeigt einen kaum messbaren Abbau in Gegenwart von Sauerstoff.

**Tabelle: Wiederfindungsmengen an Piperazin bzw. N-Hydroxyethyl-piperazin**

| Wiederfindung des Aktivators im Stresstest | Beginn des Stresstets C_{0 Tage} [%] | Nach 28 Tagen Stresstest C_{28 Tage} [%] |
|---|---|---|
| Piperazin | 100 | 94 |
| N-Hydroxyethyl-piperazin | 100 | 99 |

## Patentansprüche

1. Absorptionsmittel zum Entfernen saurer Gase aus einem Fluidstrom, umfassend eine wässrige Lösung
(A) eines Alkalimetallsalzes einer N,N-Di-C₁-C₄-alkyl-aminocarbonsäure und
(B) N-Hydroxyethylpiperazin.

2. Absorptionsmittel nach Anspruch 1, wobei die wässrige Lösung 2 bis 5 kmol / m³ Alkalimetallsalz der N,N-Di-C₁-C₄₋alkyl-aminocarbonsäure und 0,1 bis 1,5 kmol / m³ N-Hydroxyethylpiperazin umfasst.

3. Absorptionsmittel nach Anspruch 1 oder 2, wobei das Alkalimetallsalz der N,N-Di-C₁-C₄-alkyl-aminocarbonsäure Kalium-N,N-dimethylglycinat ist.

4. Verfahren zum Entfernen saurer Gase aus einem Fluidstrom, bei dem man den Fluidstrom mit einem Absorptionsmittel nach einem der vorhergehenden Ansprüche in Kontakt bringt.

5. Verfahren nach Anspruch 4, wobei der Fluidstrom sauerstoffhaltig ist.

6. Verfahren nach Anspruch 5, wobei der Fluidstrom der Oxidation organischer Substanzen entstammt.

7. Verfahren nach Anspruch 4, wobei der Fluidstrom durch im Wesentlichen anaerobe Zersetzung von Biomasse erhalten ist.

8. Verfahren nach einem der Ansprüche 4 bis 7, wobei man das beladene Absorptionsmittel durch
a) Erwärmung,
b) Entspannung,
c) Strippen mit einem inerten Fluid
oder eine Kombination zweier oder aller dieser Maßnahmen regeneriert.

## Claims

1. An absorption medium for removing acid gases from a fluid stream comprising an aqueous solution
(A) of an alkali metal salt of an N,N-di-C₁-C₄-alkylaminocarboxylic acid and
(B) N-hydroxyethylpiperazine.

2. The absorption medium according to claim 1, wherein the aqueous solution comprises 2 to 5 kmol/m³ of alkali metal salt of the N,N-di-C₁-C₄-alkylamino-carboxylic acid and 0.1 to 1.5 kmol/m³ of N-hydroxyethylpiperazine.

3. The absorption medium according to claim 1 or 2, wherein the alkali metal salt of the N,N-di-C₁-C₄-alkylaminocarboxylic acid is potassium N,N-dimethylglycinate.

4. A process for removing acid gases from a fluid stream, in which the fluid stream is contacted with an absorption medium according to any one of the preceding claims.

5. The process according to claim 4, wherein the fluid stream is oxygen-comprising.

6. The process according to claim 5, wherein the fluid stream originates from the oxidation of organic substances.

7. The process according to claim 4, wherein the fluid stream is obtained by essentially anaerobic decomposition of biomass.

8. The process according to any one of claims 4 to 7, wherein the loaded absorption medium is regenerated by
a) heating,
b) expansion,
c) stripping with an inert fluid
or a combination of two or all of these measures.

## Revendications

1. Agent d'absorption pour l'élimination de gaz acides d'un courant fluide, comprenant une solution aqueuse
(A) d'un sel de métal alcalin d'un acide N,N-di-alkyle en C₁-C₄-aminocarboxylique et
(B) de N-hydroxyéthylpipérazine.

2. Agent d'absorption selon la revendication 1, dans lequel la solution aqueuse comprend 2 à 5 kmol/m³ de sel de métal alcalin de l'acide N,N-di-alkyle en C₁-C₄-aminocarboxylique et 0,1 à 1,5 kmol/m³ de N-hydroxyéthylpipérazine.

3. Agent d'absorption selon la revendication 1 ou 2, dans lequel le sel de métal alcalin de l'acide N,N-dialkyle en C₁-C₄-aminocarboxylique est le N,N-diméthylglycinate de potassium.

4. Procédé d'élimination de gaz acides d'un courant fluide, selon lequel le courant fluide est mis en contact avec un agent d'absorption selon l'une quelconque des revendications précédentes.

5. Procédé selon la revendication 4, dans lequel le courant fluide contient de l'oxygène.

6. Procédé selon la revendication 5, dans lequel le courant fluide provient de l'oxydation de substances organiques.

7. Procédé selon la revendication 4, dans lequel le courant fluide est obtenu par décomposition essentiellement anaérobie d'une biomasse.

8. Procédé selon l'une quelconque des revendications 4 à 7, dans lequel l'agent d'absorption chargé est régénéré par
a) chauffage,
b) détente,
c) extraction avec un fluide inerte
ou une combinaison de deux ou de l'ensemble de ces mesures.
